(19) Europäisches Patentamt
European Patent Office
Office européen des brevets

(11) **EP 2 330 416 B1**

(12) **EUROPÄISCHE PATENTSCHRIFT**

(45) Veröffentlichungstag und Bekanntmachung des Hinweises auf die Patenterteilung:
**08.02.2012 Patentblatt 2012/06**

(51) Int Cl.:
*G01N 33/00* (2006.01)      *G01N 33/497* (2006.01)

(21) Anmeldenummer: **09177650.0**

(22) Anmeldetag: **01.12.2009**

(54) **Verfahren zur Verifizierung einer elektrochemischen Substanz in einer Gasprobe**

Method for verifying an electrochemical substance in a gas sample

Procédé de vérification d'une substance électrochimique dans un échantillon de gaz

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**08.06.2011 Patentblatt 2011/23**

(73) Patentinhaber: **Dräger Safety AG & Co. KGaA 23560 Lübeck (DE)**

(72) Erfinder: **Stock, Burkhard 23564, Lübeck (DE)**

(74) Vertreter: **UEXKÜLL & STOLBERG Patentanwälte Beselerstrasse 4 22607 Hamburg (DE)**

(56) Entgegenhaltungen:
**DE-A1- 4 344 196      DE-A1- 19 514 215 US-A- 5 048 321**

EP 2 330 416 B1

## Beschreibung

Die Erfindung betrifft ein Verfahren zur Verifizierung einer elektrochemischen Substanz in einer Gasprobe nach Anspruch 1.

Verfahren zur Bestimmung von Kenngrößen einer elektrochemischen Substanz in einer Gasprobe sind aus der DE 43 44 196 C2 und der US5048321 bekannt geworden.

Bei dem bekannten Verfahren wird der Anteil der elektrochemischen Substanz in der Gasprobe aus einer Integration über der zwischen einer Bezugslinie und einem Maximalwert einer physikalischen Messgröße eingeschlossenen Gesamtfläche berechnet. Für die Berechnung werden dabei Teilflächen als Teilintegrale des Gesamtintegrals der Gesamtfläche benutzt, aus denen verschiedene Kenngrößen der nachzuweisenden Substanz wie z. B. der Konzentrationsanteil in der Gasprobe oder die Stoffart der Substanz bestimmbar sind.

Eine Erkennung der elektrochemischen Substanz erfolgt auf dem Vergleich von den Teilflächen, die von dem Kurvenverlauf einer elektrischen Messgröße der Messzelle und der Zeit gebildet werden. Ein Vorhandensein weiterer chemischer Substanzen führt zu einem langsameren den Ablauf der elektrochemischen Prozesse. Im Ergebnis wird das Profil des elektrischen Messwertes über die Zeit verändert. Damit verändert sich auch das Verhältnis der Flächenanteile.

Bei oben bekannten Verfahren hat sich herausgestellt, dass eine Alterung der Messzelle sowie eine Verringerung der Temperatur zu einer Erhöhung des Innenwiderstandes der Messzelle führen. Als Folge davon verlaufen die elektrochemischen Prozesse verlangsamt.

Bei der bekannten Messzelle ändert sich somit der Kurvenverlauf des Messsignals mit zunehmender Alterung der Messzelle. So wird das Kurvenprofil im Verlauf der Gebrauchszeit flacher und breiter. Eine ähnliche Änderung des Kurvenverlaufes des Messsignals stellt sich auch bei dem Vorhandensein weiterer elektrochemischer Substanzen ein. Somit kann ein flacheres Kurvenprofil aus einer Alterung oder einer niedrigeren Temperatur der elektrochemischen Messzelle oder aber auch aus dem Vorhandensein weiterer elektrochemischer Substanzen resultieren.

Der Erfindung liegt somit die Aufgabe zugrunde, ein Auswerteverfahren für einen elektrischen Messwert einer elektrochemischen Messzelle derart zu verbessern, dass der Konzentrationsanteil der nachzuweisenden Substanz mit hoher Langzeitstabilität über die Gebrauchszeit der Messzelle zuverlässig bestimmbar ist.

Diese Aufgabe wird durch ein Verfahren zur Verifizierung einer elektrochemischen Substanz in einer Gasprobe mit den Merkmalen des Anspruchs 1 gelöst.

In den davon abhängigen Ansprüchen sind vorteilhafte Ausgestaltungen und Weiterbildungen der Erfindung angegeben.

Die Lösung der Aufgabe erfolgt dadurch, dass die Steigungen des Verlaufs eines sich zeitlich verändernden elektrischen Messwertes, der eine von einer Bezugslinie bis zu einem Maximalwert ansteigende und auf die Bezugslinie wieder zurückgehende Kennlinie erzeugt, in einem Bereich nach Überschreitung des Maximalwertes analysiert werden. Dies erfolgt dadurch, dass in einem ersten Verfahrensschritt ein erstes und ein zweites Intervall im Bereich der Kennlinie nach Überschreitung des Maximalwertes festgelegt wird, wobei das erste Intervall den Kennlinienbereich in der Nähe des Maximalwertes und das zweite Intervall den Kennlinienbereich in der Nähe der Bezugslinie umfasst. Weiter wird ein Steigungsverhältnis der Kennlinie des ersten und zweiten Intervalls ermittelt und im Anschluss daran mit einem Referenzwert des Steigungsverhältnisses des ersten und zweiten Intervalls dieser elektrochemischen Substanz verglichen.

In vorteilhafter Weise lässt sich dadurch die Zuverlässigkeit des Messergebnisses einer elektrochemischen Messzelle verbessern. Insbesondere kann damit in Anwendung der elektrochemischen Messzelle in einem Atemalkoholmessgerät die Zuverlässigkeit des Meßergebnisses erhöht werden. Dies gilt insbesondere für den Einfluss von weiteren elektrochemischen Substanzen auf die zu bestimmenden elektrochemische Substanz.

Durch die deutliche Verbesserung des erfindungsgemäßen Verfahrens können damit die Messergebnisse von elektronischen Handmessgeräten zur Atemalkoholmessung die auf einem elektrochemischen Nachweisverfahren beruhen, auch für gerichtliche Gutachten verwendet werden. Es kann ausgeschlossen werden, dass das Messergebnis nicht durch äußere Einflüsse wie beispielsweise einen Gerätedefekt oder aufgrund von Fremdsubstanzen verfälscht ist.

In einer weiteren vorteilhaften Ausbildung des erfindungsgemäßen Verfahrens kann sich das erste Intervall von einem Prozentanteil des Maximalwertes von etwa 90% bis zu einem Prozentanteil des Maximalwertes von etwa 80% erstrecken. Ferner kann sich das zweite Intervall in vorteilhafter Weise von einem Prozentanteil des Maximalwertes von etwa 20% bis zu einem Prozentanteil des Maximalwertes bis etwa 10% erstrecken.

Die nachzuweisende elektrochemische Substanz umfasst insbesondere für die Atemalkoholmessung Ethanol. Der Referenzwert der Steigungsverhältnisse des ersten und zweiten Intervalls der zu bestimmenden elektrochemischen Substanz wird während der Erstkalibrierung des elektrochemischen Sensors ermittelt.

Ferner sind Mittel zur Speicherung des Referenzwertes vorgesehen.

In einer weiteren vorteilhaften Ausbildung des erfindungsgemäßen Verfahrens wird das Messergebnis für ungültig definiert, falls der Referenzwert von dem ermittelten Wert der Steigungsverhältnisse der Kennlinie des ersten und zweiten Intervalls abweicht. Alternativ hierzu kann ein zulässiger Grenzwert vorgesehen sein und eine Verwerfung des Messergebnisses bei einer Überschreitung von dem zulässigen Grenzwert erfolgen.

In einer weiteren Alternative des erfindungsgemäßen Verfahrens kann eine Korrektur des Messergebnisses erfolgen, falls der Referenzwert von dem ermittelten Wert aus dem Steigungsverhältnis der Kennlinien des ersten und zweiten Intervalls abweicht oder einen zulässigen Grenzwert überschreitet. Eine Korrektur des Messergebnisses kann dabei durch eine Reduzierung des prozentualen Anteils der elektrochemischen Substanz in der Gasprobe erfolgen. Eine Abweichung des Referenzwertes von dem ermittelten Wert aus den Steigungsverhältnissen der Kennlinie des ersten und zweiten Intervalls kann in vorteilhafter Weise visuell oder akustisch signalisiert werden.

Das erfindungsgemäße Verfahren kann in einer Vorrichtung zur Alkoholmessung insbesondere in einem Atemalkoholmessgerät verwendet werden.

Die vorliegende Erfindung wird mit Bezug auf die beigefügten Zeichnungen detaillierter erläutert, wobei gleiche Bezugszeichen gleiche Figuren bezeichnen.

In der Zeichnung gilt:

Fig. 1:  Darstellung des zeitlichen Verlaufes des elektrischen Messwertes eines elektrochemischen Sensors einer Gasprobe mit Ethanol,

Fig. 2:  Darstellung der zeitlichen Verläufe der elektrischen Messwerte eines elektrochemischen Sensors einer Gasprobe mit Ethanol und einer Gasprobe eines Ethanol/Methanolgemisches,

Fig. 3:  schematische Darstellung einer Vorrichtung zur Durchführung des Verfahrens.

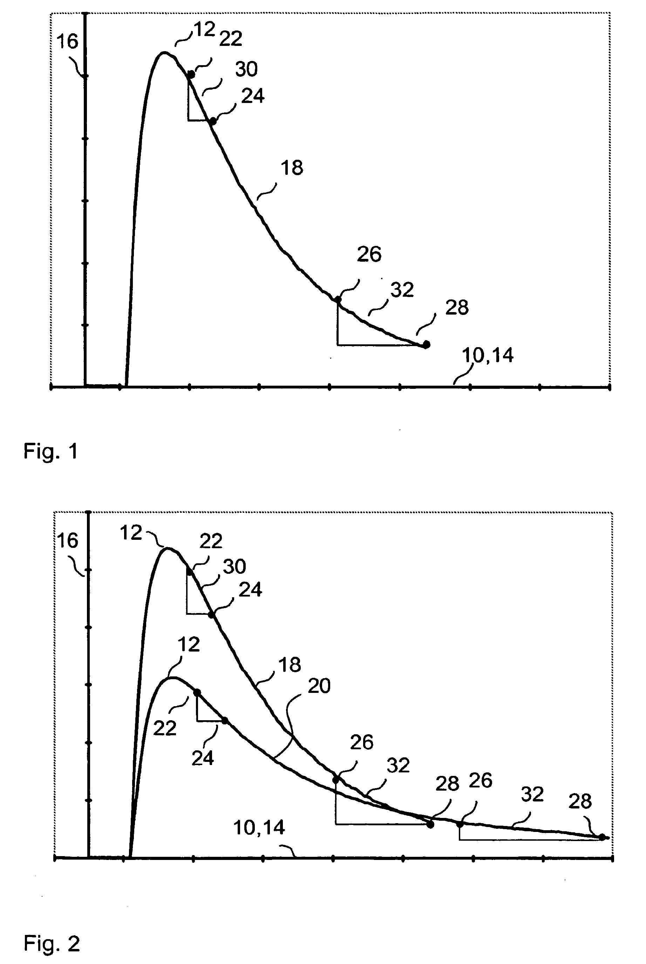

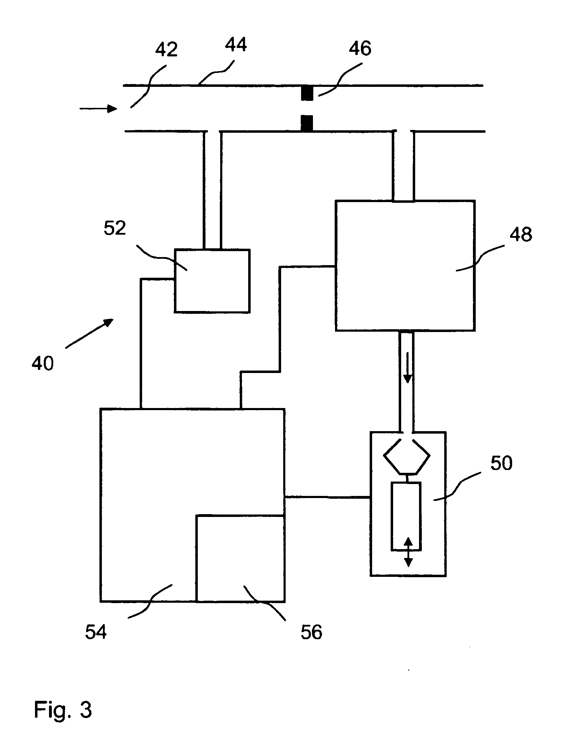

In Fig. 1 ist der Verlauf eines Stromwertes eines elektrochemischen Sensors 48 (dargestellt in Fig. 3) nach Beaufschlagung einer Gasprobe 42 mit Ethanol entlang einer Zeitachse 14 und einer Stromachse 16 dargestellt. Der sich zeitlich verändernde Stromwert des elektrochemischen Sensors 48 steigt von einer Bezugslinie 10 steil bis zu einem Maximalwert 12 an und fällt von dem Maximalwert 12 exponentiell zu der Bezugslinie 10 wieder ab. Die in der Fig. 1 dargestellte Kennlinie 18 entspricht der Strom / Zeit- Kennlinie von Ethanol. Eine experimentelle Analyse ergab, dass es sich bei dem Kennlinienbereich zwischen dem Maximalwert 12 und der Bezugslinie 10 um eine Überlagerung mehrerer Exponentialfunktionen mit unterschiedlichen Abfallzeiten handelt, die durch eine mehrstufige Umsetzung von Ethanol bis hin zu $CO_2$ und Wasser hervorgerufen werden. Ferner zeigt es sich, dass bei einer Alterung des elektrochemischen Sensors 48 sowie bei niedrigeren Temperaturen die elektrochemischen Prozesse langsamer erfolgen. Die prinzipielle Form der Strom / Zeit - Kennlinie 18 bleibt jedoch erhalten.

In der Fig. 2 ist zu der Strom/Zeit - Kennlinie 18 von Ethanol eine weitere Strom / Zeit- Kennlinie 20 aus einem Gemisch von Ethanol und Methanol dargestellt. Dieses Gemisch aus Ethanol und Methanol verfügt über andere Reaktionsstufen mit einem anderen Zeitverhalten. Die Strom / Zeit - Kennlinie 20 des Gemisches ist mit der des Ethanols nicht mehr affin. Ein Abfall der Strom / Zeit - Kennlinie von Methanol nach der Überschreitung des Maximalwertes 12 fällt deutlich flacher aus, da die Oxidation des Methanols über andere Stufen erfolgt als die von Ethanol.

In der Anwendung des erfindungsgemäßen Verfahrens werden in einem ersten Schritt ein erstes Intervall 30 und ein zweites Intervall 32 im Bereich der Kennlinie nach Überschreiten des Maximalwertes 12 festgelegt. Das erste Intervall 30 wird dabei von einem Stromwert 22 zu einem Zeitpunkt 1 und einem Stromwert 24 zu einem Zeitpunkt 2 gebildet. Das zweite Intervall wird von einem Stromwert 26 zu einem Zeitpunkt 3 und einem Stromwert 28 zu einem Zeitpunkt 4 gebildet. In vorteilhafter Weise entspricht der Stromwert 22 einem Prozentanteil von etwa 90% des Maximalwertes 12 und der Stromwert 24 einem Prozentanteil von etwa 80% des Maximalwertes 12. Der Stromwert 26 entspricht in etwa 20% des Maximalwertes 12, und der Stromwert 28 entspricht in etwa 10% des Maximalwertes 12.

In einem weiteren Verfahrensschritt wird das Steigungsverhältnis der Strom /Zeit - Kennlinie 18 von Ethanol des ersten und zweiten Intervalls 30, 32 ermittelt. Die Steigung des ersten Intervalls 30 ergibt sich entsprechend der Formel:

$$F1 = (Stromwert\ 22 - Stromwert\ 24) / (Zeitpunkt\ 1 - Zeitpunkt\ 2)$$

Die Steigung des zweiten Intervalls 32 ergibt sich analog dazu entsprechend der Formel

$$F2 = (Stromwert\ 26 - Stromwert\ 28) / (Zeitpunkt\ 3 - Zeitpunkt\ 4)$$

Somit ergibt sich ein Verhältnis der Steigungen S entsprechend der Formel:

$$S = S1 / S2.$$

In einem nachfolgenden Schritt wird das somit ermittelte Verhältnis der Steigungen S mit einem Referenzwert $S_{ref}$ des Steigungsverhältnisses des ersten und zweiten Intervalls 30, 32 dieser elektrochemischen Substanz verglichen gemäß der Formel:

$$K = S / S_{ref}.$$

Der Vergleichswert K ist unabhängig von dem Alter des elektrochemischen Sensors 48. Der Vergleichswert K ist nur abhängig von der in dem elektrochemischen Sensor 48 umgesetzten elektrochemischen Substanz.
Der Referenzwert $S_{ref}$ des Steigungsverhältnisses des ersten und zweiten Intervalls 30, 32 der zu bestimmenden elektrochemischen Substanz wird während einer ersten Kalibrierung des elektrochemischen Sensors 48, im Allgemeinen nach dessen Herstellung, ermittelt und im Anschluss gespeichert. Erfolgt eine Beaufschlagung des elektrochemischen Sensors 48 mit einer Gasprobe 42 von Ethanol, so ergibt sich ein Vergleichswert von K = 1. Wird zu der Gasprobe 42 von Ethanol die elektrochemische Substanz Methanol zugemischt, so wird der Vergleichswert K entsprechend ansteigen. Die Strom /Zeit - Kennlinie 20 wird entsprechend verändert, verglichen mit der Strom /Zeit- Kennlinie 18 von Ethanol.
Die in der Figur 2 beispielhaft dargestellte Kennlinie 20 von Ethanol und Methanol entspricht einer Mischung aus 120 ppm Ethanol und 60 ppm Methanol. Das Stromsignal fällt nach dem Maximum 12 des Stromwertes entsprechend deutlich flacher ab, da die Umsetzung von Methanol wesentlich langsamer erfolgt als die Umsetzung von Ethanol. Der Kennlinienanteil vor dem Maximalwert 12 wird von Ethanol bestimmt, wobei der Anteil nach dem Maximalwert 12 von Methanol bestimmt wird. Aufgrund der abweichenden Strom / Zeit - Kennlinie 20 von der Strom / Zeit - Kennlinie 18 steigt der Vergleichswert K entsprechend an. Für das dargestellte Beispiel entspricht der Vergleichswert K = 1,6.
Bei einer Abweichung des Vergleichswertes K von 1 kann nun das Messergebnis für ungültig definiert werden. Alternativ hierzu kann auch ein Grenzwert vorgesehen werden, beispielsweise von 1,2. Bei einer Überschreitung des Grenzwertes wird das Messergebnis entsprechend für ungültig definiert. In einer weiteren Alternative kann das Messergebnis mit einem ansteigenden Vergleichswert K entsprechend korrigiert werden, wobei beispielsweise ein prozentualer Anteil einer Begrenzung entsprechend der folgenden Formel erfolgen kann:

$$\text{Messergebnis} = \text{Messergebnis} * (1 - a * (K - 1))$$

Bei einem Vorhandensein von ausschließlich Ethanol in der Gasprobe ergibt sich ein Vergleichswert K von 1. Mit einem steigenden Anteil einer weiteren elektrochemischen Substanz, beispielsweise Methanol, wird auch der Vergleichswert K entsprechend ansteigen. Das Messergebnis wird entsprechend reduziert, wobei ein Maß der Reduzierung mit einem Faktor a einstellbar ist. Mit einem Faktor a = 0,3 ergibt sich für das vorstehend beschriebene Beispiel:

$$\text{Messergebnis} = \text{Messergebnis} * (1 - 0,3 * (1,6 - 1))$$

$$\text{Messergebnis} = \text{Messergebnis} * 0,82$$

Das erfindungsgemäße Verfahren stellt somit aufgrund der Unabhängigkeit von dem Alterungsprozess des elektrochemischen Sensors 48 sowie der Temperatur der Messung ein zuverlässiges Verfahren zur Verifizierung einer elektrochemischen Substanz in einer Gasprobe dar.
Die Figur 3 zeigt schematisch den Aufbau einer Vorrichtung zur Durchführung des Verfahrens am Beispiel eines Atemalkoholmessgerätes 40.
Die Atemluft eines Probanden strömt als Gasprobe 42 durch ein Mundstück 44. Die Gasprobe 42 erzeugt dabei einen Druckabfall an einer Blende 46. Die Gasprobe 42 gelangt sowohl auf einen Drucksensor 52 als auch auf den

**EP 2 330 416 B1**

elektrochemischen Sensor 48. Der Drucksensor 52 misst bei einer Probengabe des Probanden kontinuierlich den Druck und errechnet daraus das von dem Probanden abgegebene Volumen der Gasprobe 42. Nach Abgabe eines definierten Volumens der Gasprobe 42 startet eine Steuereinheit 54 eine Pumpe 50. Dadurch wird die Gasprobe 42 in den elektrochemischen Sensor 48 gezogen. Aus dem Stromsignal des elektrochemischen Sensors 48 wird von der Steuereinheit 54 eine entsprechende Atemalkoholkonzentration ermittelt. In einer Speichereinheit 56, die als integraler Bestandteil der Steuereinheit 54 ausgeführt sein kann, ist der Referenzwert der Steigungsverhältnisse des ersten und zweiten Intervalls 30, 32 der zu bestimmenden elektrochemischen Substanz abgelegt.

BEZUGSZEICHENLISTE

10 -    Bezugslinie

12 -    Maximalwert

14 -    Zeitachse

16 -    Stromachse

18 -    Strom/Zeit -Kennlinie von Ethanol

20 -    Strom/Zeit -Kennlinie eines Gemischs aus Ethanol/Methanol

22 -    Stromwert zum Zeitpunkt 1

24 -    Stromwert zum Zeitpunkt 2

26 -    Stromwert zum Zeitpunkt 3

28 -    Stromwert zum Zeitpunkt 4

30 -    erstes Intervall

32 -    zweites Intervall

40 -    Atemalkoholmessgerät

42 -    Gasprobe

44 -    Mundstück

46 -    Blende

48 -    elektrochemischer Sensor

50 -    Pumpe

52 -    Drucksensor

54 -    Steuereinheit

56 -    Speichereinheit

**Patentansprüche**

**1.**  Verfahren zur Verifizierung einer elektrochemischen Substanz in einer Gasprobe (42), welche in einem elektrochemischen Sensor (48) einen sich zeitlich verändernden elektrischen Messwert mit einer von einer Bezugslinie (10) bis zu einem Maximalwert (12) ansteigenden und auf die Bezugslinie (10) wieder zurückgehenden Kennlinie (18, 20) erzeugt, aus dem in einer Auswerteschaltung der Anteil der elektrochemischen Substanz in der Gasprobe (42) durch die folgenden Schritte bestimmt wird:

a) Festlegung eines ersten und eines zweiten Intervalls (30, 32) im Bereich der Kennlinie (18, 20) nach Überschreitung des Maximalwertes (12), wobei das erste Intervall (30) den Kennlinienbereich in der Nähe des Maximalwertes (12) und das zweite Intervall (32) den Kennlinienbereich in der Nähe der Bezugslinie (10) umfasst,

b) Ermittlung des Steigungsverhältnisses (K) des ersten und zweiten Intervalls (30, 32) und

c) Vergleich mit einem Referenzwert des Steigungsverhältnisses (Kref) des ersten und zweiten Intervalls (30, 32) dieser elektrochemischen Substanz.

2. Verfahren nach Anspruch 1, **dadurch gekennzeichnet, dass** sich das erste Intervall (30) von einem Prozentanteil des Maximalwertes von etwa 90% bis zu einem Prozentanteil des Maximalwertes von etwa 80% erstreckt.

3. Verfahren nach einem der vorhergehenden Ansprüche, **dadurch gekennzeichnet, dass** sich das zweite Intervall (32) von einem Prozentanteil des Maximalwertes von etwa 20% bis zu einem Prozentanteil des Maximalwertes von etwa 10% erstreckt.

4. Verfahren nach einem der vorhergehenden Ansprüche, **dadurch gekennzeichnet, dass** der elektrische Messwert einen Stromwert umfasst.

5. Verfahren nach einem der vorhergehenden Ansprüche, **dadurch gekennzeichnet, dass** die elektrochemische Substanz Ethanol umfasst.

6. Verfahren nach einem der vorhergehenden Ansprüche, **dadurch gekennzeichnet, dass** der Referenzwert des Steigungsverhältnisses (Kref) des ersten und zweiten Intervalls (30, 32) der zu bestimmenden elektrochemischen Substanz während der Erstkalibrierung des elektrochemischen Sensors (48) ermittelt wird.

7. Verfahren nach Anspruch 6, **dadurch gekennzeichnet, dass** Mittel zur Speicherung (56) des Referenzwertes (Kref) vorgesehen sind.

8. Verfahren nach einem der vorhergehenden Ansprüche, **dadurch gekennzeichnet, dass** ein Messergebnis für ungültig definiert wird, falls der Referenzwert (Kref) von dem im Verfahrensschritt b) ermittelten Wert abweicht.

9. Verfahren nach einem der Ansprüche 1 bis 7, **dadurch gekennzeichnet, dass** das Messergebnis für ungültig definiert wird, falls eine Abweichung des Referenzwertes (Kref) von dem im Verfahrensschritt b) ermittelten Wert einen Grenzwert überschreitet.

10. Verfahren nach einem der Ansprüchen 1 bis 7. **dadurch gekennzeichnet, dass** eine Korrektur des Messergebnisses erfolgt, falls der Referenzwert (Kref) von dem im Verfahrensschritt b) ermittelten Wert abweicht oder eine Abweichung des Referenzwertes (Kref) von dem im Verfahrensschritt b) ermittelten Wert einen Grenzwert überschreitet.

11. Verfahren nach Anspruch 10, **dadurch gekennzeichnet, dass** ein prozentualer Anteil der elektrochemischen Substanz in der Gasprobe (42) reduziert wird.

12. Verfahrens nach einem der vorhergehenden Ansprüche, **dadurch gekennzeichnet, dass** eine Abweichung des Referenzwertes (Kref) von dem im Verfahrensschritt b) ermittelten Wert signalisiert wird.

13. Verwendung des Verfahrens nach den Ansprüchen 1 bis 12 in einem Atemalkoholmessgerät (40).

**Claims**

1. Method for verifying an electrochemical substance in a gas sample (42) which, in an electrochemical sensor (48), generates a measured electric value changings over time according to a characteristic curve (18, 20) rising from a reference line (10) to a maximum (12) and dropping to the reference line (10) again, from which measured electric value a percentage of the electrochemical substance in the gas sample (42) is determined in an analysis circuit by the following steps:

a) setting of a first and second interval (30, 32) in the range of the characteristic curve (18, 20) after the maximum (12) has been exceeded, wherein the first interval (30) comprises the range of the characteristic curve in the vicinity of the maximum and the second interval (32) comprises the range of the characteristic in the vicinity of the reference line (10),
b) determining a ratio of the slopes (K) of the first and second intervals (30, 32), and
c) comparing the ratio of the slopes of the first and second intervals (30, 32) of this electrochemical substance with a reference value (Kref).

2. Method according to claim 1, **characterized in that** the first interval (30) extends from a percentage of the maximalum equaling about 90% to a percentage of the maximalum equaling about 80%.

3. Method according to any of the preceding claims, **characterized in that** the second interval (32) extends from a percentage of the maximum equaling about 20% to a percentage of the maximum equaling about 10%.

4. Method according to any of the preceding claims, **characterized in that** the measured electric value comprises a current value.

5. Method according to any of the preceding claims, **characterized in that** the electrochemical substance comprises ethyl alcohol.

6. Method according to any of the preceding claims, **characterized in that** the reference value of the ratio of the slopes (Kref) of the first and second intervals (30, 32) of the electrochemical substance to be determined is determined during the first calibration of the electrochemical sensor (48).

7. Method according to claim 6, **characterized in that** a means for storing (56) the reference value (Kref) is provided.

8. Method according to any of the preceding claims, **characterized in that** a measuring result is defined as being invalid if the reference value (Kref) deviates from the value determined in step b).

9. Method according to any of the claims 1 to 7, **characterized in that** the measuring result is defined is being invalid if a deviation of the reference value (Kref) from the value determined in step b) exceeds a limit value.

10. Method according to any of the claims 1 to 7, **characterized in that** a correction of the measuring result is performed if the reference value (Kref) deviates from the value determined in step b) or if a deviation of the reference value (Kref) from the value determined in step b) exceeds a limit value.

11. Method according to claim 10, **characterized in that** a percentage of the electrochemical substance in the gas sample (42) is reduced.

12. Method according to any of the preceding claims, **characterized in that** a deviation of the reference value (Kref) from the value determined in step b) is signaled.

13. Use of the method according to claims 1 to 12 in an alcohol measuring device (40).

**Revendications**

1. Procédé pour vérifier une substance électrochimique contenue dans un prélèvement gazeux (42), donnant dans un capteur électrochimique (48) une valeur de mesure électrique qui varie avec le temps et qui, partant d'une ligne de référence (10) augmente jusqu'à une valeur maximale (12) pour revenir à la ligne de référence (10) en décrivant une courbe caractéristique (18, 20), un circuit d'exploitation définissant à partir de la valeur mesurée le taux de la substance électrochimique dans le prélèvement gazeux (42), le procédé présentant les étapes suivantes :

a) fixation d'un premier et d'un second intervalle (30, 32) dans la zone de la courbe caractéristique (18, 20) après passage à la valeur maximale (12), le premier intervalle (30) englobant la zone des lignes caractéristiques située à proximité de la valeur maximale (12) tandis que le second intervalle (32) englobe la zone des lignes caractéristiques se trouvant à proximité de la ligne de référence (10),

b) établissement du rapport (K) des pentes du premier et du second intervalle (30) 32),

c) comparaison à une valeur de référence du rapport des pentes (Kref) du premier et du second intervalle (30, 32) de cette substance électrochimique.

2. Procédé selon la revendication 1, **caractérisé en ce que** le premier intervalle (30) s'étend d'un pourcentage de la valeur maximale d'environ 90 % à un pourcentage de cette valeur d'environ 80 %.

3. Procédé selon une des revendications précédentes, **caractérisé en ce que** le second intervalle (32) s'étend d'un pourcentage de la valeur maximale d'environ 20 % à un pourcentage de cette valeur d'environ 10 %.

4. Procédé selon une des revendications précédentes, **caractérisé en ce que** la valeur électrique de mesure comprend une valeur de courant.

5. Procédé selon une des revendications précédentes, **caractérisé en ce que** la substance électrochimique comprend de l'éthanol.

6. Procédé selon une des revendications précédentes, **caractérisé en ce que** la valeur de référence du rapport de pentes (Kref) du premier et du second intervalles (30, 32) de la substance électrochimique à déterminer est établie pendant le premier calibrage du capteur électrochimique (48).

7. Procédé selon la revendication 6, **caractérisé en ce que** des moyens sont prévus pour la mémorisation (56) de la valeur de référence (Kref).

8. Procédé selon une des revendications précédentes, **caractérisé en ce qu'**un résultat de mesure est considéré comme non valable dans le cas où la valeur de référence (Kref) s'écarte de la valeur déterminée à l'étape (b) du procédé.

9. Procédé selon une des revendications 1 à 7, **caractérisé en ce que** le résultat de mesure est considéré comme non valable dans le cas où l'écart entre la valeur de référence (Kref) et la valeur déterminée à l'étape (b) du procédé dépasse une valeur limite.

10. Procédé selon une des revendications 1 à 7, **caractérisé en ce que** le résultat de mesure subit une correction dans le cas où la valeur de référence (Kref) s'écarte de la valeur établie à l'étape (b) du procédé ou dans le cas où l'écart de la valeur de référence (Kref) dépasse la valeur déterminée à l'étape (b) du procédé.

11. Procédé selon la revendication 10, **caractérisé en ce qu'**est réduit un pourcentage de la substance électrochimique dans le prélèvement gazeux (42).

12. Procédé selon une des revendications précédentes, **caractérisé en ce qu'**est signalé un écart de la valeur de référence (Kref) par rapport à la valeur établie à l'étape (b) du procédé.

13. Utilisation du procédé selon les revendications 1 à 12, dans un appareil de mesure de l'alcool contenu dans l'air expiré (40).

Fig. 1

Fig. 2

Fig. 3

**IN DER BESCHREIBUNG AUFGEFÜHRTE DOKUMENTE**

**In der Beschreibung aufgeführte Patentdokumente**

- DE 4344196 C2
- US 5048321 A